# EUROPÄISCHE PATENTSCHRIFT

(11) **EP 0 765 646 B1**
(45) Veröffentlichungstag und Bekanntmachung des Hinweises auf die Patenterteilung: **06.12.2000**
(21) Anmeldenummer: 95114084.7
(22) Anmeldetag: 08.09.1995
(51) Int. Cl.: A61F 2/60, A61F 2/80

(54) **Unterschenkelprothese**
Lower leg prosthesis
Prothèse pour la jambe inférieure

(43) Veröffentlichungstag der Anmeldung: 02.04.1997
(73) Patentinhaber: Otto Bock Orthopädische Industrie Besitz- und Verwaltungs-Kommanditgesellschaft, 37115 Duderstadt (DE)
(72) Erfinder: Becker, Karl, 37115 Duderstadt 24 (DE); Krukenberg, Manfred, 37115 Duderstadt (DE)
(74) Vertreter: Gramm, Werner, Prof. Dipl.-Ing.

(56) Entgegenhaltungen:
- EP-A- 0 019 612
- WO-A-92/08425
- WO-A-93/15696
- WO-A-94/24965
- DE-U- 9 419 210
- US-A- 1 868 303
- US-A- 5 139 523
- US-A- 5 156 629

## Beschreibung

Die Erfindung betrifft eine Unterschenkelprothese mit einem steifen, schalenförmigen, ein geschlossenes distales Ende aufweisenden Außenschaft und einem herausnehmbar darin eingesetzten flexiblen Innenschaft, der ebenfalls ein geschlossenes distales Ende und eine über ein Ventil aufblasbare Luftkammer aufweist und dazu bestimmt und geeignet ist, einen Stumpf eines Prothesenträgers allseitig zu umschließen.

Eine derartige Ausführungsform läßt sich beispielsweise der US 5,156,629A entnehmen. Der mit dem aus einem Polymer bestehenden Innenschaft versehene Stumpf wird von oben in den nur oben offenen Hohlraum des Außenschaftes eingeführt. Das für den Tragekomfort wesentliche Problem ergibt sich aus der Volumenänderung des Stumpfes, die aus dem dynamischen Flüssigkeitsvolumen im Körper des Prothesenträgers resultiert und sich von einem Tag zum anderen ändern kann. Aus diesem Grunde muß der Außenschaft bzw. sein Hohlraum nicht unbedingt an die Außenkontur des Stumpfes angepaßt werden. Vielmehr sollte insbesondere im Bereich des distalen Stumpfendes ein eine Stumpfausdehnung ermöglichender Hohlraum verbleiben. Hierdurch könnte aber der Prothesenträger seine Prothese verlieren, wenn durch Atrophie oder Volumenschwankung des Stumpfes der Außenschaft scheinbar zu groß wird. Es entsteht dann eine schlechte Paßform in Verbindung mit einer verstärkten Pumpwirkung. Das Gehen wird mühsam und ist häufig von unangenehmen Geräuschen und Schmerzen begleitet. Dieses Problem wird bei der eingangs beschriebenen Ausführungsform nur teilweise durch das Aufblasen des Innenschaftes mit Luft gelöst. So fehlt insbesondere ein zuverlässiger Kraft- oder Formschluß zwischen Innen- und Außenschaft.

Die US 5,139,523 A offenbart ebenfalls eine Unterschenkelprothese mit einem steifen, ein geschlossenes distales Ende aufweisenden Außenschaft, der im wesentlichen aus einem starren zylindrischen, durch Längsstäbe gebildeten und in Kunststoff eingebetteten Käfig besteht, der mit seinem unteren Rand in dem massiven, halbkugelförmig ausgebildeten distalen Endabschnitt des Außenschaftes verankert ist, der an seinem unteren Ende ein Kupplungsteil zur Verbindung des Außenschaftes mit der Prothese aufweist. Innerhalb des Außenschaftes, der eine geschlossene zylindrische innere Kunststoff-Mantelfläche aufweist, ist eine aufblasbare Blase festgelegt, die eine das distale Ende des Außenschaft-Hohlraumes abdeckende Luftkammer aufweist, die in pneumatischer Verbindung steht mit einer zweiten Luftkammer, die an der inneren Mantelfläche des Außenschaftes anliegt und sich über dessen volle axiale Länge erstreckt. Das Aufblasen dieser Blase erfolgt mit Hilfe einer in das distale Ende des Außenschaftes integrierten Luftpumpe, die über eine durch das genannte distale Ende hindurchgeführte Druckleitung an die erste der beiden vorstehend genannten Luftkammern der Blase angeschlossen ist, deren Innenluftdruck abgelassen werden kann durch ein manuell von außen, ebenfalls in das distale Außenschaft-Ende integriertes Ablaßventil.

Der Erfindung liegt die Aufgabe zugrunde, die eingangs beschriebene Unterschenkelprothese hinsichtlich ihres Tragekomforts zu verbessern.

Ausgehend von der Unterschenkelprothese gemäß der eingangs beschriebenen Ausführungsform wird diese Aufgabe erfindungsgemäß durch folgende zusätzliche Merkmale gelöst:
a) Der Innenschaft ist als Silikonschaft ausgebildet, der so weich ist, daß er über den genannten Stumpf des Prothesenträgers abrollbar ist;
b) die Luftkammer des Innenschaftes umschließt nur dessen distales Ende allseitig;
c) am distalen Ende des Innenschaftes ist ein Steckzapfen befestigt, der über eine Durchgangsbohrung mit der Luftkammer in luftaustauschender Verbindung steht;
d) im distalen Ende des Außenschaftes ist eine dem Steckzapfen angepaßte und ihn lösbar aufnehmende Einstecköffnung vorgesehen;
e) an die Einstecköffnung ist ein Schlauch angeschlossen, dessen nach außen geführtes Schlauchende an ein Auslaßventil und eine Pumpe angeschlossen ist.

Diese Lösung läßt einerseits einen geregelten Volumenausgleich zwischen Stumpf und Außenschaft zu, wobei die erzielte breitflächige Verteilung der auf den Stumpf einwirkenden Kräfte den Komfort erhöht und die Aktivität des Prothesenträgers steigert. Der durch das Aufpumpen der Luftkammer entstehende Druck verteilt sich in die Weichteile, die dann zusätzlich rund um die Knochen verschoben werden und dadurch die Polsterwirkung der Weichteile erhöhen. Der Kontakt zwischen Stumpf und Prothese wird optimal; er erhöht die Koordination (Lenkung der Prothese) und verringert Traumas. Die erfindungsgemäße Luftkammer als integraler Bestandteil des Innenschaftes schmiegt sich vollumfänglich an das distale Stumpfende an und paßt sich dabei jeder Stumpfform an. Aufgrund des weichen, hautverträglichen Silikons tritt eine Verhornung der Haut nicht auf; Scherkräfte werden weitgehend vom Silikon aufgenommen. Daher kann der Innenschaft unmittelbar auf der Haut getragen werden. Die Haftung (Suspension) erfolgt teilweise durch Kraftschluß und teilweise durch Formschluß. Um zu verhindern, daß durch das Aufpumpen der Luftkammer der Innenschaft und damit der Stumpf aus dem Außenschaft gedrückt wird, ist es zweckmäßig, wenn im Außenschaft im Bereich des distalen Stumpfendes über die Höhe bzw. Länge der Luftkammer ein hinreichender Ausdehnungsraum zur Verfügung steht, wobei in dem darüber liegenden Bereich eine zumindest teilweise angenähert zylindrische Ausbildung des Außenschaft-Hohlraumes zweckmäßig ist. Das Differenzvolumen zwischen Stumpf und Außenschaft läßt sich dann durch die Luftkammer vollständig ausfüllen. Erreicht werden Total-Kontakt und Endbelastung und damit ein angenähert idealer hydrostatischer Druck innerhalb des Stumpfes.

Erfindungswesentlich ist aber die zusätzliche Fixierung des Innenschaftes im Außenschaft durch eine kraft- und formschlüssige Verbindung des distalen Endes des Innenschaftes, also des distalen Endes der Luftkammer mit dem distalen Ende des Außenschaftes. Die erfindungsgemäß vorgesehene Steckzapfenverbindung garantiert eine zuverlässige Verbindung zwischen Innen- und Außenschaft sowie die unveränderbare Position des Innenschaftes im Außenschaft und gibt zudem die Möglichkeit, die Pumpe mit dem Schlauch zum Aufpumpen der Luftkammer getrennt von dem Innenschaft unmittelbar im Außenschaft anzuordnen. Der Innenschaft muß diese Bauteile also nicht mehr aufweisen, so daß es sich anbietet, durch verschiedene Silikonschäfte ein Modulsystem zu bilden. Über nach Größen unterschiedliche Module wird der Stumpf des Patienten vermessen, um dann aus dem Silikon-Innenschaftsystem einen hinsichtlich der Größe geeigneten Silikonschaft auszuwählen.

Die Verbindung des Innenschaftes mit dem Außenschaft erfolgt selbsttätig beim Einsteigen des bereits mit dem Silikonschaft versehenen Stumpfes in den Außenschaft. Dabei ist es zweckmäßig, wenn der Steckzapfen über seine Länge mit Gummiringen bestückt ist, denen in der Einstecköffnung ringförmige Rastrillen zugeordnet sind. Durch Austauschen der Gummiringe und/oder durch Veränderung der Anzahl der Gummiringe läßt sich die Friktion der Steckverbindung auf die Bedürfnisse des Patienten einstellen. Darüberhinaus dienen die Luftringe auch zur Abdichtung des Luftsystems.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:
- **Figur 1**: einen Längsschnitt durch eine Unterschenkelprothese;
- **Figur 2**: in einer Explosionsdarstellung die Einbauteile der Unterschenkelprothese gemäß Figur 1;
- **Figur 3**: in gegenüber Figur 2 vergrößertem Maßstab im Längsschnitt ein Einsteckteil;
- **Figur 4**: ein Klemmstück in einer Schnittdarstellung gemäß Figur 3;
- **Figur 5**: einen Längsschnitt durch eine Aufnahme;
- **Figur 6**: in perspektivischer Darstellung eine Wechselaufnahme und
- **Figur 7**: in perspektivischer Darstellung ein Eingußteil.

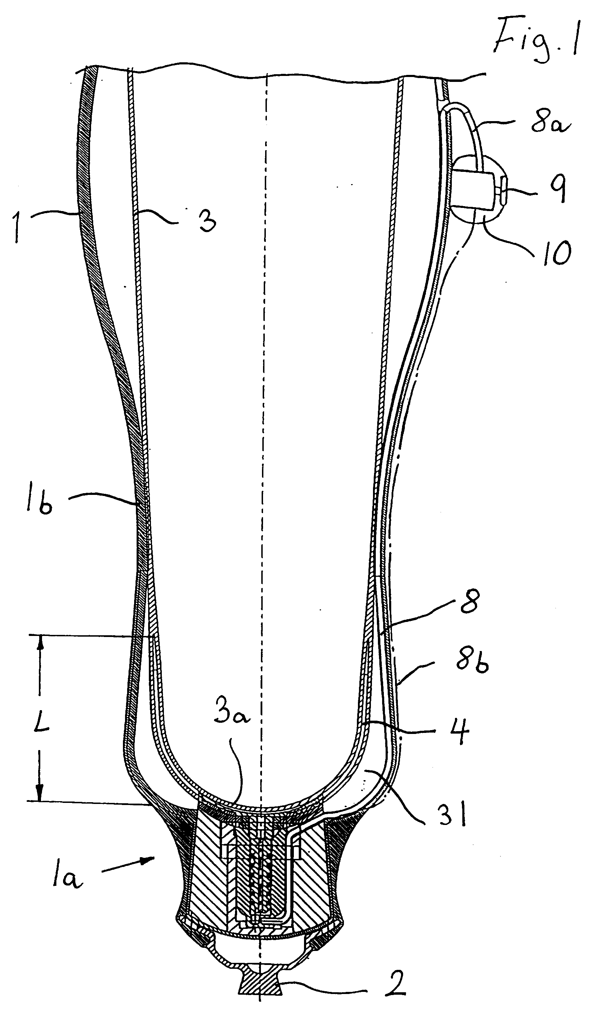

Die in Figur 1 dargestellte Unterschenkelprothese weist einen steifen, schalenförmigen Außenschaft 1 mit einem geschlossenen, einen Anschluß 2 für ein Modulsystem aufweisenden distalen Ende 1a sowie einen herausnehmbar darin eingesetzten flexiblen Innenschaft 3 auf, der ebenfalls ein geschlossenes distales Ende 3a aufweist. Dieser Innenschaft 3 ist als weicher, über den Stumpf des Prothesenträgers abrollbarer, den Stumpf allseitig umschließender und an ihm anliegender Silikonschaft ausgebildet, der in seinem distalen Bereich doppellagig ausgebildet ist und hier eine geschlossene Luftkammer 4 bildet, die das distale Stumpfende allseitig umschließt und sich nur über eine kleine Teillänge L des Innenschaftes 3 erstreckt.

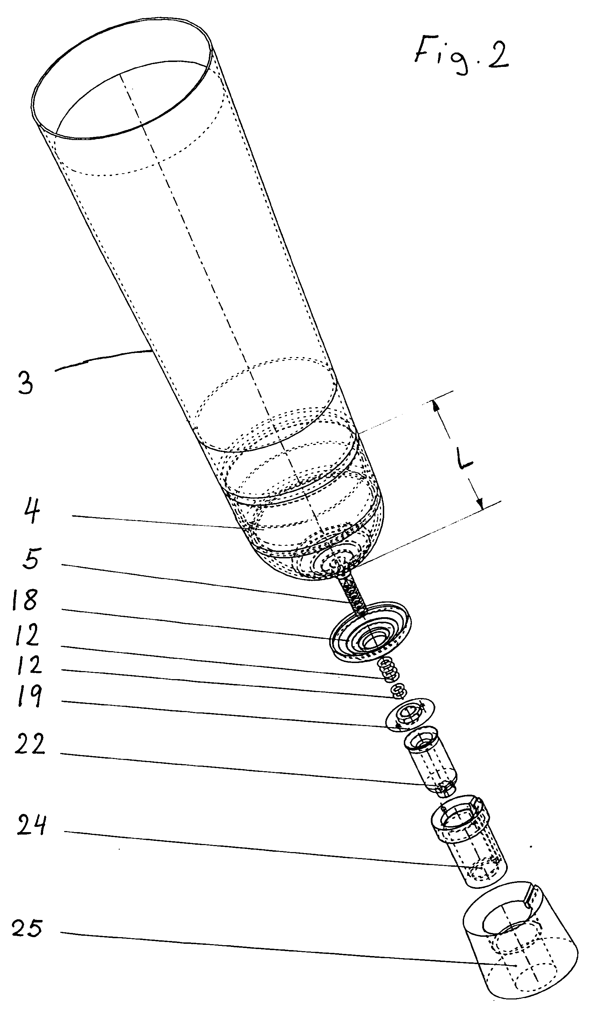

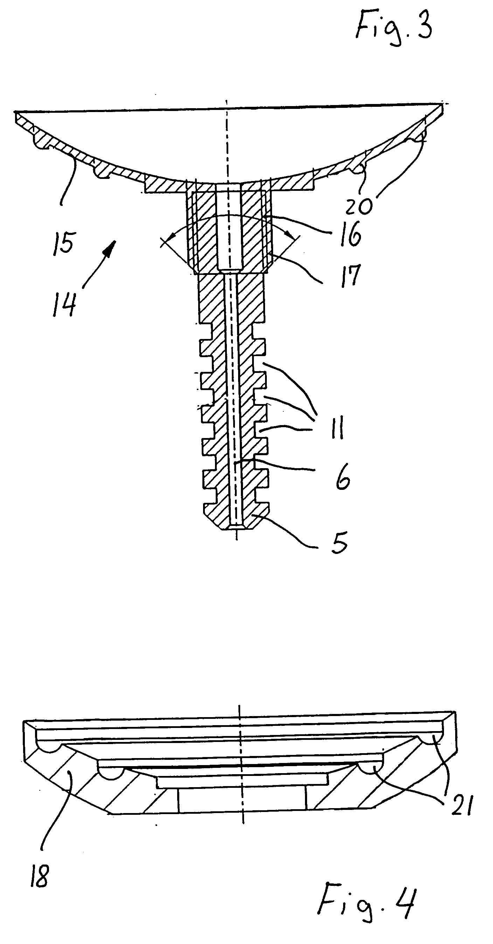

Am distalen Ende 3a des Innenschaftes 3 ist ein Steckzapfen 5 befestigt (siehe Figur 2 und 3), der über eine Durchgangsbohrung 6 mit der Luftkammer 4 in luftaustauschender Verbindung steht. Im distalen Ende 1a des Außenschaftes 1 ist eine dem Steckzapfen 5 angepaßte und ihn lösbar aufnehmende Einstecköffnung 7 vorgesehen, an die ein Schlauch 8 angeschlossen ist (siehe Figur 1), dessen nach außen geführtes Schlauchende 8a an ein Auslaßventil 9 und eine Pumpe 10 angeschlossen ist. Dabei ist das Schlauchende 8a durch den Außenschaft 1 hindurch nach außen geführt; das Auslaßventil 9 sowie die Pumpe 10 sind an der Außenseite des Außenschaftes 1 befestigt. Die Schlauchführung kann jedoch gemäß der strichpunktierten Linie 8b auch weitgehend außerhalb des Außenschaftes 1 liegen.

Figur 3 läßt erkennen, daß der Steckzapfen 5 sechs über seine Länge verteilt angeordnete Ringnuten 11 aufweist, die jeweils zur auswechselbaren Aufnahme eines Gummiringes 12 (siehe Figur 2) dienen. Diesen sechs Gummiringen 12, die in eingelegtem Zustand etwas über die Mantelfläche des Steckzapfens 5 hinausragen, sind in der Einstecköffnung 7 ringförmige Rastrillen 13 zugeordnet.

Zur Befestigung des Steckzapfens 5 am distalen Ende 3a des Innenschaftes 3 ist ein Einsteckteil 14 vorgesehen, das mit einer kalottenförmigen Halteplatte 15 innerhalb der Luftkammer 4 angeordnet ist und deren distale Wandung 4a mit einer Anschlußbuchse 16 durchdringt, die mit einem Außengewinde 17 versehen ist. Der vorzugsweise aus Aluminium bestehende Steckzapfen 5 ist in die Anschlußbuchse 16 eingepreßt oder aber auch eingeschraubt. Auf die Anschlußbuchse 16 ist ein von außen gegen das distale Ende 3a des Innenschaftes 3 anliegendes Klemmstück 18 geschoben und mittels einer auf die Anschlußbuchse 16 aufgeschraubten Spannmutter 19 (siehe Figur 2) gegenüber der Halteplatte 15 verspannbar. Um eine rutschfeste Einspannung der distalen Wandung 4a der Luftkammer 4 sicherzustellen, weist die Halteplatte 15 zwei Ringwulste 20 auf, denen im Klemmstück 18 zwei Ringnuten 21 zugeordnet sind (siehe Figur 4).

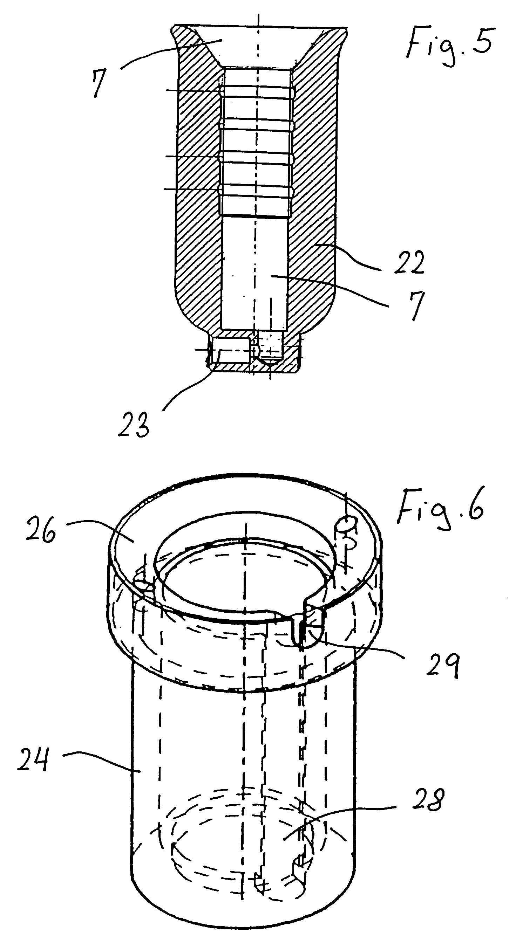

Die Einstecköffnung 7 stellt die zentrische Längsbohrung einer Aufnahme 22 dar (siehe Figur 5) und steht mit einer in deren distalen Ende vorgesehenen Bohrung 23 in Verbindung, an die der Schlauch 8 angeschlossen ist.

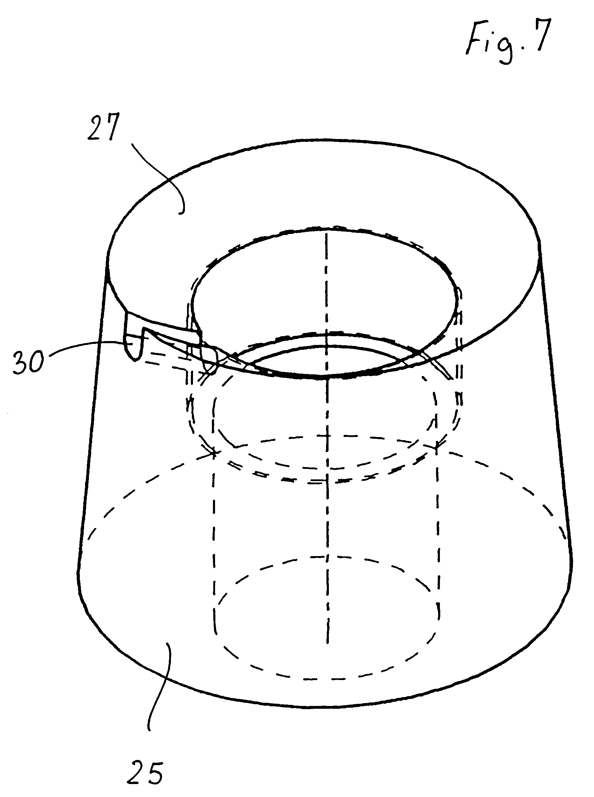

Die Aufnahme 22 ist umschlossen von einer topfförmig ausgebildeten Wechselaufnahme 24, die in ein in das distale Ende 1a des Außenschaftes 1 einlaminiertes Eingußteil 25 (siehe Figur 7) gesteckt ist. Die Wechselaufnahme 24 weist eine ringförmige, konisch oder ballig ausgebildete Auflagefläche 26 auf, auf der sich ein abgerundeter Kopf der Spannmutter 19 abstützt. Diese Auflagefläche 26 liegt bündig mit einer sie umschließenden Ringfläche 27 des Eingußteils 25.

Zur Verlegung des Schlauches 8 ist in der Innenwandung der Wechselaufnahme 24 eine achsparallele, zum Innenraum hin offene Nut 28 vorgesehen, die in einer in der Auflagefläche 26 vorgesehenen Quernut 29 mündet, die mit einer entsprechend ausgebildeten Quernut 30 in der Ringfläche 27 des Eingußteils 25 fluchtet.

Halteplatte 15 mit Anschlußbuchse 16, Klemmstück 18, die z.B. als Zweilochmutter ausgebildete Spannmutter 19, Aufnahme 22, Wechselaufnahme 24 und Eingußteil 25 können aus Kunststoff ausgebildet sein.

Der Patient legt den Innenschaft 3 am Stumpf an, bevor er in den Außenschaft 1 der Prothese steigt und hierbei durch Einführen des Steckzapfens 5 in die Einstecköffnung 7 eine selbsttätige Einrastung und damit Verbindung des Innenschaftes 3 mit dem Außenschaft 1 bewirkt. Dabei läßt sich die Friktion über die Gummiringe 12 des Steckzapfens 5 auf die Bedürfnisse des Patienten einstellen. Zur Erzielung einer höheren Friktion werden Gummiringe mit einer höheren Shore-Härte gewählt, so daß sich eine festere Friktionsverbindung ergibt. Umgekehrt wird zur Erzielung einer niedrigeren Friktion für die Gummiringe eine niedrigere Shore-Härte gewählt, oder aber die Anzahl der Gummiringe wird reduziert, wodurch das Ein- und Aussteigen für den Patienten erleichtert wird. Die Gummiringe 12 dienen zugleich aber auch als Luftabdichtung.

Nach dem Einsteigen mit dem Innenschaft 3 in den Außenschaft 1 kann der Patient das Volumen im distalen Abschnitt des Innenschaftes 3 durch Aufpumpen der Luftkammer 4 selbst bestimmen. Die Luftverbindung erfolgt von der Pumpe 10 über den Schlauch 8, die Bohrung 23, die Einstecköffnung 7 und die Durchgangsbohrung 6 im Steckzapfen 5.

Sollte beim Anziehen der Prothese der Steckzapfen 5 nicht von vornherein vollständig in die Einstecköffnung 7 eingeführt werden, kann der Patient durch Aufpumpen der Luftkammer 4 einen Druck nach distal auf die Steckverbindung geben, so daß die Friktion der Gummiringe 12 in der Aufnahme 22 immer optimal ausgenutzt wird, und der mit den Gummiringen 12 bestückte Steckzapfen 5 immer die gleiche Position in der Aufnahme 22 hat.

Um zu verhindern, daß durch das Aufpumpen der Luftkammer 4 der Innenschaft 3 und damit der Stumpf aus dem Außenschaft 1 gedrückt wird, ist im Außenschaft 1 im Bereich des distalen Stumpfendes über die Höhe bzw. Länge L der Luftkammer 4 ein hinreichender Ausdehnungsraum 31 vorgesehen, an den sich nach oben ein zumindest teilweise angenähert zylindrischer Abschnitt 1b des Außenschaftes 1 anschließt. Dabei beträgt die Teillänge L in einer bevorzugten Ausführungsform etwa 7 cm.

## Patentansprüche

1. Unterschenkelprothese mit einem steifen, schalenförmigen, ein geschlossenes distales Ende (1a) aufweisenden Außenschaft (1) und einem herausnehmbar darin eingesetzten flexiblen Innenschaft (3), der ebenfalls ein geschlossenes distales Ende (3a) und eine über ein Ventil (9, 10) aufblasbare Luftkammer (4) aufweist und dazu bestimmt und geeignet ist, einen Stumpf eines Prothesenträgers allseitig zu umschließen,
**gekennzeichnet** durch folgende Merkmale:
a) der Innenschaft (3) ist als Silikonschaft ausgebildet, der so weich ist, daß er über den genannten Stumpf des Prothesenträgers abrollbar ist;
b) die Luftkammer (4) des Innenschaftes (3) umschließt nur dessen distales Ende (3a) allseitig;
c) am distalen Ende (3a) des Innenschaftes (3) ist ein Steckzapfen (5) befestigt, der über eine Durchgangsbohrung (6) mit der Luftkammer (4) in luftaustauschender Verbindung steht;
d) im distalen Ende (1a) des Außenschaftes (1) ist eine dem Steckzapfen (5) angepaßte und ihn lösbar aufnehmende Einstecköffnung (7) vorgesehen;
e) an die Einstecköffnung (7) ist ein Schlauch (8) angeschlossen, dessen nach außen geführtes Schlauchende (8a) an ein Auslaßventil (9) und eine Pumpe (10) angeschlossen ist.

2. Unterschenkelprothese nach Anspruch 1, **dadurch gekennzeichnet,** daß der Steckzapfen (5) über seine Länge mit Gummiringen (12) bestückt ist, denen in der Einstecköffnung (7) ringförmige Rastrillen (13) zugeordnet sind.

3. Unterschenkelprothese nach Anspruch 2, **dadurch gekennzeichnet,** daß die Gummiringe (12) auswechselbar angeordnet sind.

4. Unterschenkelprothese nach Anspruch 1, 2 oder 3, **dadurch gekennzeichnet,** daß der Steckzapfen (5) mit einem Einsteckteil (14) verbunden ist, das mit einer kalottenförmigen Halteplatte (15) innerhalb der Luftkammer (4) angeordnet ist und deren distale Wandung (4a) mit einer Anschlußbuchse (16) zur Befestigung des Steckzapfens (5) durchdringt.

5. Unterschenkelprothese nach Anspruch 4, **dadurch gekennzeichnet,** daß auf die mit einem Außengewinde (17) versehene Anschlußbuchse (16) ein Klemmstück (18) geschoben ist, das von außen gegen das distale Ende (3a) des Innenschaftes (3) anliegt und mittels einer auf die Anschlußbuchse (18) aufgeschraubten Spannmutter (19) gegenüber der Halteplatte (15) verspannbar ist.

6. Unterschenkelprothese nach Anspruch 4 und 5, **dadurch gekennzeichnet,** daß die Halteplatte (15) oder das Klemmstück (18) zumindest eine Ringwulst (20) aufweist, der im Klemmstück (18) oder der Halteplatte (15) eine Ringnut (21) zugeordnet ist.

7. Unterschenkelprothese nach einem der vorhergehenden Ansprüche, **dadurch gekennzeichnet,** daß die Einstecköffnung (7) die zentrische Längsbohrung in einer Aufnahme (22) darstellt und mit einer in deren distalen Ende vorgesehenen Bohrung (23) in Verbindung steht, an die der Schlauch (8) angeschlossen ist.

8. Unterschenkelprothese nach Anspruch 7, **dadurch gekennzeichnet,** daß die Aufnahme (22) innerhalb einer topfförmig ausgebildeten Wechselaufnahme (24) angeordnet ist, die in ein in das distale Ende (1a) des Außenschaftes (1) einlaminiertes Eingußteil (25) gesteckt ist.

9. Unterschenkelprothese nach Anspruch 5 und 8, **dadurch gekennzeichnet,** daß die Wechselaufnahme (24) eine ringförmige, konisch oder ballig ausgebildete Auflagefläche (26) für den abgerundeten Kopf der Spannmutter (19) aufweist.

10. Unterschenkelprothese nach einem der vorhergehenden Ansprüche, **dadurch gekennzeichnet,** daß das Schlauchende (8a) durch den Außenschaft (1) hindurch nach außen geführt und das Auslaßventil (9) sowie die Pumpe (10) an der Außenseite des Außenschaftes (1) befestigt sind.

## Claims

1. Lower leg prosthesis having a rigid, shell-shaped outer shaft (1), which has a closed distal end (1a), and a flexible inner shaft (3), which is removably inserted into the outer shaft and likewise has a closed distal end (3a) and an air chamber (4) inflatable by means of a valve (9, 10) and is intended and suitable for enclosing a stump of a prosthesis wearer on all sides,
**characterized** by the following features:
a) the inner shaft (3) takes the form of a silicone shaft, which is soft enough to be rollable over the said stump of the prosthesis wearer;
b) the air chamber (4) of the inner shaft (3) encloses only the latter's distal end (3a) on all sides;
c) fastened to the distal end (3a) of the inner shaft (3) is a plug-in pin (5), which is connected by a through-bore (6) in an air-exchanging manner to the air chamber (4);
d) provided in the distal end (1a) of the outer shaft (1) is an insertion opening (7) which is adapted to, and releasably receives the plug-in pin (5);
e) connected to the insertion opening (7) is a tube (8), of which the tube end (8a) conveyed to the outside is connected to an outlet valve (9) and a pump (10).

2. Lower leg prosthesis according to claim 1, **characterized in** that the plug-in pin (5) is equipped over its length with rubber rings (12), with which annular detent grooves (13) in the insertion opening (7) are associated.

3. Lower leg prosthesis according to claim 2, **characterized in** that the rubber rings (12) are disposed in an exchangeable manner.

4. Lower leg prosthesis according to claim 1, 2 or 3, **characterized in** that the plug-in pin (5) is connected to an insertion part (14), which is disposed with a spherical cap-shaped retaining plate (15) inside the air chamber (4) and penetrates the latter's distal wall (4a) with a connecting bush (16) for fastening of the plug-in pin (5).

5. Lower leg prosthesis according to claim 4, **characterized in** that slipped onto the connecting bush (16), which is provided with an external thread (17), is a clamping piece (18), which is applied from the outside against the distal end (3a) of the inner shaft (3) and is braceable relative to the retaining plate (15) by means of a clamping nut (19) screwed onto the connecting bush (18).

6. Lower leg prosthesis according to claim 4 and 5, **characterized in** that the retaining plate (15) or the clamping piece (18) comprises at least one annular bead (20), with which an annular groove (21) in the clamping piece (18) or retaining plate (15) is associated.

7. Lower leg prosthesis according to one of the preceding claims, **characterized in** that the insertion opening (7) is the centrical longitudinal bore in a receiver (22) and is connected to a bore (23), which is provided in the distal end of said receiver and to which the tube (8) is connected.

8. Lower leg prosthesis according to claim 7, **characterized in** that the receiver (22) is disposed inside a pot-shaped interchangeable receiver (24), which is inserted into an encapsulated part (25) laminated into the distal end (1a) of the outer shaft (1).

9. Lower leg prosthesis according to claim 5 and 8, **characterized in** that the interchangeable receiver (24) has an annular supporting surface (26) of a conical or crowned construction for the rounded head of the clamping nut (19).

10. Lower leg prosthesis according to one of the preceding claims, **characterized in** that the tube end (8a) is conveyed outwards through the outer shaft (1), and the outlet valve (9) and the pump (10) are fastened to the outside of the outer shaft (1).

## Revendications

1. Prothèse pour la jambe inférieure, comprenant un fût extérieur (1) rigide, en forme de coque, présentant une extrémité distale fermée (1a), et un fût intérieur (3) flexible monté de manière amovible dans le fût extérieur, et qui présente également une extrémité distale fermée (3a) et une chambre à air (4) pouvant être gonflée par une valve (9, 10), le fût intérieur étant destiné et approprié pour entourer de toutes parts un moignon d'un porteur de prothèse, caractérisée par les particularités suivantes:
a) le fût intérieur (3) est réalisé sous la forme d'un fût silicone qui est si mou, qu'il peut être déroulé sur le moignon précité du porteur de prothèse ;
b) la chambre à air (4) du fût intérieur (3) n'enveloppe que l'extrémité distale du moignon de toutes parts ;
c) à l'extrémité distale (3a) du fût intérieur (3) est fixée une broche d'emboîtement (5) qui est en liaison d'échange d'air avec la chambre à air (4) par l'intermédiaire d'un alésage traversant (6);
d) une ouverture d'emboîtement (7) adaptée à la broche d'emboîtement (5) et recevant celle-ci de manière démontable est prévue dans l'extrémité distale (la) du fût extérieur (1) ;
e) à l'ouverture d'emboîtement (7) est raccordé un tuyau (8) dont l'extrémité (8a) guidée vers l'extérieur est raccordée à une valve de sortie (9) et à une pompe (10).

2. Prothèse pour la jambe inférieure selon la revendication 1, caractérisée en ce que la broche d'emboîtement (5) est équipée sur sa longueur avec des anneaux en caoutchouc (12) auxquels sont associées des rainures d'encliquetage annulaires (13) dans l'ouverture d'emboîtement (7).

3. Prothèse pour la jambe inférieure selon la revendication 2, caractérisée en ce que les anneaux en caoutchouc (12) sont disposés de manière remplaçable.

4. Prothèse pour la jambe inférieure selon la revendication 1, 2 ou 3, caractérisée en ce que la broche d'emboîtement (5) est reliée à une pièce d'emboîtement (14), qui par une plaque de retenue (15) en forme de calotte est montée à l'intérieur de la chambre à air (4) et qui par une douille de raccordement (16) pour la fixation de la broche d'emboîtement (5), traverse la paroi distale (4a) de la chambre à air (4).

5. Prothèse pour la jambe inférieure selon la revendication 4, caractérisée en ce qu'une pièce de serrage (18) enfilée sur la douille de raccordement (16) prévue avec un filetage extérieur (17), s'appuie de l'extérieur contre l'extrémité distale (3a) du fût intérieur (3) et peut être serrée par rapport à la plaque de retenue (15) au moyen d'un écrou de serrage (19) vissé sur la douille de raccordement (18).

6. Prothèse pour la jambe inférieure selon la revendication 4 ou 5, caractérisée en ce que la plaque de retenue (15) ou la pièce de serrage (18) présente au moins un bourrelet annulaire (20), auquel est associée une rainure annulaire (21) dans la pièce de serrage (18) ou dans la plaque de retenue (15).

7. Prothèse pour la jambe inférieure selon l'une des revendications précédentes, caractérisée en ce que l'ouverture d'emboîtement (7) représente l'alésage longitudinal central dans une monture (22), et est reliée avec un alésage (23) auquel le tuyau (8) est raccordé et qui est prévu dans l'extrémité distale de la monture.

8. Prothèse pour la jambe inférieure selon la revendication 7, caractérisée en ce que la monture (22) est disposée à l'intérieur d'une monture interchangeable (24) réalisée en forme de pot, qui est emboîtée dans une pièce moulée (25) enchâssée dans l'extrémité distale (1a) du fût extérieur (1).

9. Prothèse pour la jambe inférieure selon revendication 5 et 8, caractérisée en ce que la monture interchangeable (24) présente une surface d'appui annulaire (26) réalisée conique ou bombée pour la tête arrondie de l'écrou de serrage (19).

10. Prothèse pour la jambe inférieure selon l'une des revendications précédentes, caractérisée en ce que l'extrémité (8a) du tuyau est guidée vers l'extérieur à travers le fût extérieur (1), et en ce que la valve de sortie (9) ainsi que la pompe (10) sont fixées à la face extérieure du fût extérieur (1).
